# FASCICULE DE BREVET EUROPEEN

(11) **EP 3 137 901 B1**
(45) Date de publication et mention de la délivrance du brevet: **18.09.2019**
(21) Numéro de dépôt: 14720963.9
(22) Date de dépôt: 29.04.2014
(51) Int. Cl.: G01N 33/53, G01N 30/04

(54) **PROCÉDÉ DE DOSAGE D'ACIDES ORGANIQUES URINAIRES**
VERFAHREN ZUR BESTIMMUNG VON ORGANISCHEN SÄUREN IM HARN
METHOD FOR DETERMINING URINARY ORGANIC ACIDS

(43) Date de publication de la demande: 08.03.2017
(73) Titulaire: SYNLAB Belgium, 6220 Heppignies (Fleurus) (BE)
(72) Inventeur: VERHOEFT, André, B-1390 Grez-doiceau (BE)
(74) Mandataire: Gevers Patents
(86) Numéro de dépôt international: PCT/EP2014/058745
(87) Numéro de publication internationale: WO 2015/165510

(56) Documents cités:
- WO-A2-99/22244
- FR-A1- 2 899 973
- US-A- 3 341 554
- US-A- 3 627 821
- US-A- 5 686 311
- LEE S.H. ET AL.: "Gas chromatographic-mass spectrometric determination of urinary oxoacids using O-(2,3,4,5,6-pentafluorobenzyl)oxime-trime thylsilyl ester derivatization and cation-exchange chromatography", J. CHROMATOGR. B, vol. 719, no. 1-2, 20 novembre 1998 (1998-11-20), pages 1-7, XP004144806,
- LIEN S.K. ET AL.: "Utilization of a deuterated derivatization agent to synthesize internal standards for gas chromatography-tandem mass spectrometry quantification of silylated metabolites", J. CHROMATOGR. A, vol. 1247, 28 mai 2012 (2012-05-28), pages 118-124, XP028498369,
- KALUZNA-CZAPLINSKA J. ET AL.: "Noninvasive urinary organic acids test to assess biochemical and nutritional individuality in autistic children", CLIN. BIOCHEM., vol. 44, no. 8, 12 février 2011 (2011-02-12), pages 686-691, XP028209461,
- LORD R.S. ET AL.: "Clinical applications of urinary organic acids. Part 2. Dysbiosis markers", ALTERNATIVE MEDICINE REVIEW, vol. 13, no. 4, 2008, pages 292-306, XP55148457,

## Description

La présente invention se rapporte à un procédé de dosage d'acides carboxyliques urinaires.

Le dosage d'acides carboxyliques urinaires peut être utilisé dans le domaine de la biologie nutritionnelle et fonctionnelle pour notamment détecter une dysbiose d'origine fongique ou bactérienne chez un individu. Une dysbiose se traduit par un déséquilibre du microbiote qui constitue la flore intestinale de l'individu. L'identification d'une dysbiose permet de sélectionner un traitement adéquat pour traiter la pathologie lorsque celle-ci est détectée. Lorsqu'un individu présente une dysbiose d'origine fongique ou bactérienne, les bactéries du tube digestif rejettent des produits de catabolisme, résorbés puis éliminés par le rein. Il a été observé que le spectre des métabolites excrétés dans les urines se modifie et conduit à l'observation d'élévations de taux de métabolites organiques urinaires. Leur dosage permet donc de mettre en évidence une dysbiose et ainsi de pouvoir administrer le traitement adéquat à l'individu souffrant d'un déséquilibre de la flore microbienne.

Le dosage des métabolites organiques urinaires permet d'établir un profil qui met en lumière des éléments différentiels par rapport à des profils de référence qui ne présentent pas de dysbiose. Cette mise en évidence d'écart permet de diagnostiquer une dysbiose d'origine fongique ou bactérienne. Ainsi, une approche thérapeutique ciblée peut être appliquée sur l'individu concerné. Après traitement de la dysbiose, le procédé de dosage de métabolites organiques urinaires peut à nouveau être utilisé pour évaluer un retour de profil à la normale à savoir une restauration du microbiote soit de la flore intestinale.

Les espèces organiques urinaires qui constituent un des facteurs déterminants pour évaluer une augmentation inhabituelle d'excrétions de métabolites et ainsi permettre le diagnostic d'une dysbiose sont les acides carboxyliques urinaires. Par conséquent, il est nécessaire de pouvoir les doser de manière fiable et reproductible afin de pouvoir mettre en lumière une éventuelle augmentation de leur quantité dans les urines et ainsi faciliter la détermination de l'écart différentiel précité.

L'échantillon d'urine comprend toutefois des acides carboxyliques urinaires mais aussi d'autres composants constitutifs de l'urine. Une des difficultés de la mise en oeuvre d'un procédé de dosage de ces acides carboxyliques urinaires réside dans l'isolation des espèces d'intérêts qui permettraient de déterminer la présence d'une dysbiose d'origine fongique ou bactérienne car tout échantillon d'urine contient de nombreux constituants qui ne présentent pas d'intérêt pour un tel procédé de dosage de ces acides organiques urinaires, mais qui en outre interfèrent avec les analyses.

Lee S.H. et al., J. Chromatogr. B, 1998, 719(1-2): 1-7 décrit un procédé de dosage d'acides organiques urinaires utilisant des solvants tels que MSTFA et TMCS, une phase d'isolation par évaporation, une analyse par GC-MS et la purification de l'échantillon sur colonne échangeuse d'ions.

Kaluzna-Czaplinska J. et al., 2011, 44(8): 686-691 décrit une méthode de mesure des acides organiques urinaires par GC-MS en particulier pour déterminer une dysbiose intestinale. La méthode de préparation des échantillons utilise le BSTFA.

Malheureusement, les techniques actuelles de dosage ne sont pas encore optimales pour permettre un dosage aisé, rapide, reproductible et fiable d'une grande série d'acides organiques urinaires en pathologie humaine, particulièrement dans le but de diagnostiquer une dysbiose d'origine fongique ou bactérienne.

L'invention a pour but de pallier les inconvénients de l'état de la technique en procurant un procédé de dosage d'une grande série d'acides organiques urinaires (jusque 30 différents) qui soit aisément réalisable, fiable et reproductible.

Pour résoudre ce problème, il est prévu suivant l'invention un procédé qui comprend les étapes de :
- amenée d'un volume prédéterminé d'un échantillon d'urine contenant des dérivés d'acides carboxyliques urinaires et des composants constitutifs d'urine,
- obtention d'un résidu sec desdits acides carboxyliques urinaires isolés desdits composants constitutifs d'urine,
- estérification dudit résidu sec desdits acides carboxyliques urinaires par un mélange constitué de N-Méthyl-N-triméthylsilyltrifluoroacétamide (MSTFA) et de triméthylchlorosilane (TMCS) dans de l'acétonitrile sec anhydre pour former une solution comprenant des groupements triméthylsilanesters dérivés desdits acides carboxyliques urinaires,
- dosage par chromatographie en phase gazeuse couplée à une spectrométrie de masse desdits triméthylsilanesters pour obtenir une mesure de la quantité desdits triméthylsilanesters,
- comparaison desdites quantités mesurées de triméthylsilanesters avec des valeurs prédéterminées de quantités de triméthylsilanesters correspondant à des quantités d'acides carboxyliques urinaires pour doser lesdits acides carboxyliques urinaires dans ledit volume prédéterminé.

Le traitement d'échantillon d'urine selon la présente invention comprend donc une obtention d'un résidu sec qui permet d'isoler les acides carboxyliques urinaires et les concentrer pour ensuite les estérifier en présence d'un mélange constitué de N-Méthyl-N-triméthylsilyltrifluoroacétamide (MSTFA) et de triméthylchlorosilane (TMCS) dans de l'acétonitrile sec anhydre. La réaction d'estérification consiste donc à former les triméthylsilanesters dérivés des acides carboxyliques urinaires.

Ainsi, selon la présente invention, le procédé permet de former des solutions suffisamment concentrées en acides carboxyliques urinaires qui sont isolées des composants constitutifs d'urine, lesquels ne présentent pas d'intérêts pour l'analyse par l'obtention dudit résidu sec. Cette étape du procédé selon la présente invention permet en outre d'éliminer la présence d'artéfacts de façon à obtenir un signal qui soit principalement lié à la présence des espèces devant être dosées.

Lorsque les espèces à doser sont séparées des composants constitutifs d'urine, elles sont simultanément remises en suspension et estérifiées dans un petit volume de mélange constitué de N-Méthyl-N-triméthylsilyltrifluoroacétamide (MSTFA) et de triméthylchlorosilane (TMCS) dans de l'acétonitrile sec anhydre, ce qui permet d'obtenir les triméthylsilanesters de manière concentrée dans un petit volume de solvant, pour pouvoir les identifier et les quantifier et ainsi établir un profil devant être analysé pour par exemple diagnostiquer une dysbiose. En conséquence, le procédé selon la présente invention permet de traiter l'échantillon d'urine avec une méthode de haute qualité, ce qui détermine la fiabilité et la reproductibilité du procédé de dosage utilisé.

La séparation par chromatographie en phase gazeuse dans le procédé de dosage selon la présente invention des acides carboxyliques urinaires est donc rendue aisée grâce à la formation de triméthylsilanesters, dérivant des acides carboxyliques urinaires puisque la volatilité des triméthylsilanesters est supérieure à celle des acides carboxyliques urinaires et permet de les différencier les uns des autres.

La chromatographie en phase gazeuse permet donc, de manière avantageuse, d'obtenir un spectre qui reprend un ensemble de pics distants les uns des autres correspondant chacun à différents triméthylsilanesters qui présentent donc chacun leur propre temps de rétention. Ainsi, chaque pic présente un temps de rétention spécifique à un triméthylsilanester correspondant à un acide carboxylique urinaire spécifique. Ainsi, la détermination de la quantité des triméthylsilanesters permet par la suite de déterminer la quantité des acides carboxyliques urinaires qui constituent les espèces d'intérêts.

La réaction d'estérification est un facteur déterminant pour obtenir les triméthylsilanesters qui sont nécessaires pour pouvoir appliquer la méthode chromatographique en phase gazeuse de manière efficace dont le chromatogramme sera aisément lisible et permet rapidement le dosage des acides carboxyliques urinaires. Il a été constaté que la réaction d'estérification en présence d'acétonitrile sec permet d'optimiser ladite réaction de telle façon que la formation de triméthylsilanesters est garantie et efficace. Donc, lorsque la chromatographie en phase gazeuse est appliquée, chaque triméthylsilanester correspondant à un acide carboxylique urinaire, identifié par spectrométrie de masse en tandem est séparé en fonction de son temps de rétention, ce qui permet ainsi le dosage de celui ci.

En effet, la méthode de chromatographie en phase gazeuse sépare les différents triméthylsilanesters qui sont identifiés au moyen de la spectrométrie de masse. Le résultat ainsi obtenu procure un profil de pics, lesquels sont donc identifiés et quantifiés. Chaque mesure de pic correspondant à une quantité de triméthylsilanester identifié est comparée à des quantités de triméthylsilanesters issues d'un échantillon de référence pour pouvoir réellement doser chacun des triméthylsilanesters dans le volume prédéterminé de l'échantillon.

Selon la présente invention, le dosage des acides carboxyliques urinaires individuels est obtenu de manière reproductible et aisée par l'action conjointe de l'obtention du résidu sec qui permet de concentrer l'échantillon et de l'estérifier pour pouvoir ensuite l'analyser par chromatographie gazeuse couplée à une spectrométrie de masse. Ces actions conjointes ont permis de manière très surprenante d'obtenir un dosage de ces acides carboxyliques urinaires chacun séparément.

Avantageusement, selon la présente invention, ledit mélange constitué de N-Méthyl-N-triméthylsilyltrifluoroacétamide (MSTFA) et de triméthylchlorosilane (TMCS) dans de l'acétonitrile sec anhydre est présent en une quantité comprise entre 4 et 6 %, de préférence 5 %, par rapport au volume total d'acétonitrile sec.

Avantageusement, dans lequel ladite étape d'obtention dudit résidu sec comprend les étapes de :
- traitement dudit échantillon d'urine pour former lesdits acides carboxyliques urinaires,
- isolation desdits acides carboxyliques urinaires des composant constitutifs d'urine à l'aide d'un volume prédéterminé d'une phase d'élution, et
- évaporation dudit volume prédéterminé de ladite phase d'élution pour obtenir ledit résidu sec.

L'échantillon d'urine est donc selon la présente invention traité pour former des acides carboxyliques urinaires. Ces derniers sont isolés des composants constitutifs d'urine de façon à obtenir une solution concentrée en acides carboxyliques urinaires. Il est bien entendu qu'il est possible qu'une partie des composants constitutifs d'urine soit présente dans la solution obtenue après isolation des acides carboxyliques urinaires. L'évaporation dudit volume prédéterminé permet d'encore augmenter la concentration en acides carboxyliques urinaires dans le résidu sec.

Dans une forme particulière, l'étape de traitement dudit échantillon d'urine comprend une addition d'une solution d'acide tropique diluée dans de l'acétonitrile et une addition d'acide chlorhydrique audit échantillon.

La solution d'acide tropique est une molécule spécifique qui présente une réactivité chimique et un comportement chimique proches des molécules à doser, un temps de rétention différent des molécules à doser et une concentration du même ordre de grandeur que les molécules à doser.

L'ajout d'acide chlorhydrique à l'échantillon permet d'acidifier l'urine pour obtenir des acides carboxyliques urinaires.

De préférence, l'étape de traitement comprend l'addition de 50 µL de ladite solution d'acide tropique qui présente une concentration de 20 g/L et l'addition de 800 µL d'acide chlorhydrique à 37 % audit échantillon.

Plus préférentiellement, l'étape d'obtention dudit résidu sec comprend une étape de prétraitement, précédant ladite étape de traitement, qui consiste à prétraiter l'échantillon en présence d'azide de sodium et d'acide ascorbique. L'azide de sodium permet d'éviter la prolifération bactérienne et l'acide ascorbique préserve les molécules présentes dans l'échantillon d'urine.

Avantageusement, l'étape d'isolation desdits acides carboxyliques est obtenue par adsorption sur de la terre de diatomées desdits acides carboxyliques et une élution desdits acides carboxyliques de la terre de diatomées par ledit volume prédéterminé de ladite phase d'élution contenant de l'acétate d'éthyle et du tert-butyl méthyl éther.

L'étape d'isolation, comme dit précédemment, permet d'augmenter la concentration en acides carboxyliques. Cette étape est réalisée sur une cartouche d'extraction en phase solide qui comprend une cartouche d'extraction qui est chargée en terre de diatomées sur lesquelles se fixent les acides carboxyliques urinaires. Ainsi, les acides carboxyliques urinaires sont piégés dans la cartouche d'extraction qui contient la terre de diatomée et sont séparés des composants constitutifs d'urine grâce à l'addition de la phase d'élution. La phase d'élution permet de décrocher les acides carboxyliques urinaires de la cartouche d'extraction afin d'obtenir une solution concentrée en acides carboxyliques urinaires.

De manière encore plus préférentielle, ledit volume prédéterminé de ladite phase d'élution consiste en 25mL de ladite phase d'élution.

Avantageusement, l'étape d'évaporation est réalisée sous azote à 37 °C. La durée de l'étape d'évaporation doit être suffisamment longue pour s'assurer que le résidu obtenu soit suffisamment sec.

Dans une forme de réalisation particulièrement avantageuse du procédé selon l'invention, ladite étape d'estérification est réalisée durant une période de temps allant de 20 à 40 minutes, de préférence 30 minutes dans une étuve à une température supérieure à 50 °C, de préférence de 60 °C. L'application d'une température lors de la réaction d'estérification permet de favoriser la cinétique de réaction.

D'autres formes de réalisation du procédé suivant l'invention sont indiquées dans les revendications annexées.

Est aussi décrite l' utilisation du procédé de dosage selon l'invention, ladite utilisation comprenant les étapes de :
- établissement d'un profil de données à analyser, lesdites données étant obtenues à l'issu du procédé,
- comparaison du profil de données établi avec une série de profils prédéterminés enregistrés dans un espace de stockage de données,
- détermination d'un écart différentiel entre ledit profil établi et lesdits profils prédéterminés et,
- identification d'une dysbiose d'origine fongique ou bactérienne.

L'avantage de l'utilisation du procédé de dosage suivant l'invention se situe dans la possibilité d'identifier une pathologie humaine, notamment une dysbiose d'origine fongique ou bactérienne. Cela permet de détecter un déséquilibre du microbiote d'un individu et ainsi de déterminer un traitement adéquat pour restaurer la flore intestinale.

Le procédé de dosage suivant l'invention comprend une amenée d'un volume prédéterminé d'un échantillon d'urine contenant des dérivés d'acides carboxyliques urinaires et des composants constitutifs d'urine. L'échantillon d'urine présentant un volume prédéterminé peut être prétraité par l'ajout d'azide de sodium et d'acide ascorbique.

Le volume prédéterminé de l'échantillon, de préférence prétraité, peut ensuite être mélangé à une solution d'acide tropique diluée dans de l'acétonitrile en présence d'acide chlorhydrique. La solution d'acide tropique est préférée car la molécule d'acide tropique présente une réactivité chimique et un comportement chimique proches des molécules à doser, un temps de rétention différent des molécules à doser et une concentration du même ordre de grandeur que les molécules à doser. L'acide chlorhydrique permet de former des acides carboxyliques urinaires dont leurs dérivés, les triméthylsilanesters, peuvent être dosés.

Le mélange qui comprend donc des composants constitutifs d'urine, des acides carboxyliques urinaires, la solution d'acide tropique et de l'acide chlorhydrique est versé dans une cartouche d'extraction en phase solide chargée en terre de diatomées. La cartouche a la particularité de présenter une grande surface d'adsorption pour les molécules présentes dans l'échantillon d'urine, notamment les acides carboxyliques urinaires.

Le but de cette étape est d'isoler efficacement les espèces d'intérêts pour le dosage, soit principalement les acides carboxyliques urinaires. La première étape est l'accrochage des acides carboxyliques urinaires sur la cartouche d'extraction en phase solide chargée en terres de diatomées. La deuxième étape consiste à décrocher les acides carboxyliques urinaires à l'aide d'un solvant d'élution qui est de préférence constitué d'acétate d'éthyle et du tert-butyl méthyl éther. Ainsi, l'isolation principalement des acides carboxyliques urinaires est effectuée.

Une évaporation du solvant d'élution contenue dans la solution comprenant les acides carboxyliques urinaires est réalisée jusqu'à l'obtention d'un résidu sec concentré en acides carboxyliques urinaires.

Le résidu sec est estérifié par l'ajout d'un mélange constitué de N-Méthyl-N-triméthylsilyltrifluoroacétamide (MSTFA) et de triméthylchlorosilane (TMCS) dans de l'acétonitrile sec anhydre. La proportion de MSTFA/TMCS étant de 100/1. Le mélange MSTFA/TMCS représente environ 5 % du volume total d'acétonitrile. La réaction d'estérification permet de former des triméthylsilanesters qui sont des espèces plus volatiles que les acides carboxyliques urinaires. Pour cette raison, les triméthylsilanesters sont préférés car le dosage nécessite l'utilisation d'une chromatographie en phase gazeuse.

Pour que la réaction d'estérification soit efficace et puisse permettre la formation des triméthylsilanesters, il a été observé que l'utilisation d'acétonitrile sec est un facteur déterminant pour le bon déroulement de la réaction. De plus, la réaction d'estérification doit pouvoir être favorable afin de fournir les espèces devant être dosées. La cinétique de la réaction d'estérification est augmentée par chauffage.

La solution obtenue après estérification peut être mélangée dans de l'acétonitrile afin que les triméthylsilanesters soient dosés par chromatographie en phase gazeuse couplée à une spectrométrie de masse.

Le dosage consiste en la séparation des espèces à doser de type triméthylsilanesters. Un profil comprenant différents pics caractéristiques de chaque espèce est obtenu. Chaque espèce à doser présente un temps de rétention qui lui est propre. L'identification et la quantification de chaque pic sont réalisées en mesurant la surface sous le pic pour déterminer la quantité de triméthylsilanesters.

Le dosage des acides carboxyliques urinaires dans le volume prédéterminé de l'échantillon d'urine est réalisé en comparant les quantités mesurées des triméthylsilanesters avec des valeurs prédéterminées de quantités de triméthylsilanesters correspondant à des quantités d'acides carboxyliques urinaires.

Le procédé de dosage suivant l'invention permet de mesurer les quantités en mmol/mol de créatinine et peut être utilisé pour diagnostiquer une dysbiose d'origine fongique ou bactérienne. L'utilisation du procédé consiste alors à établir un profil de données issu d'un échantillon d'urine d'un individu. L'analyse consiste à comparer le profil de l'individu avec une série de profils d'individus qui ne présentent pas de dysbiose ou de pathologie particulière.

Ainsi, la comparaison des profils permet de mettre en évidence, si le cas se présente, un écart différentiel correspondant à une augmentation du taux d'acides organique urinaires, ce qui résulte d'une présence probable d'une dysbiose d'origine fongique ou bactérienne.

### EXEMPLE 1

Un mélange constitué de 99 % en volume de N-Méthyl-N-triméthylsilyltrifluoroacétamide (MSTFA) et de 1 % en volume de triméthylchlorosilane (TMCS) est préparé.

Dans un tube en verre (100mm x 16mm), 4 mL d'urine, 50 µL d'une solution d'acide tropique (20 g/L) ; Fluka®, 93540) et 800 µL d'acide chlorhydrique à 37 % sont mélangés pour former une solution comprenant les composants constitutifs d'urine, la solution d'acide tropique, la solution d'acide chlorhydrique et des d'acides carboxyliques urinaires qui doivent être dosés. La solution est vortexée et ensuite versée dans une cartouche d'extraction en phase solide, par exemple de type ChemElut. La cartouche utilisée est prévue pour contenir un volume d'échantillon de 5 mL et comprend une cartouche d'extraction en phase solide qui est chargée en terres de diatomées. La cartouche en terres de diatomées fournit une surface d'adsorption suffisante pour adsorber les molécules à doser, soit les acides carboxyliques urinaires. Après 10 minutes de percolation, les molécules à doser, les acides carboxyliques urinaires, sont adsorbées sur la terre de diatomées.

Le décrochage des acides carboxyliques urinaires est réalisé au moyen de 25 mL d'une phase d'élution qui est ajoutée au moyen d'une dispensette. Une aiguille est positionnée à l'extrémité de la cartouche d'extraction en phase solide afin de récupérer la totalité de l'éluât constitué d'acétate d'éthyle et de tert-butyl méthyl éther dans un tube en verre contenant un col avec un pas-de-vis. Ainsi, les acides carboxyliques urinaires sont décrochés de la terre de diatomées.

L'évaporation du solvant d'élution est réalisée dans un évaporateur sous azote durant 2-3 heures à 38 °C et permet d'obtenir un résidu sec.

Une réaction d'estérification est poursuivie sur le résidu sec en ajoutant 50 µL du mélange MSTFA/TMCS et 850 µL d'acétonitrile sec dans le tube pour former des triméthylsilanesters. Ainsi, le mélange MSTFA/TMCS représente environ 5 % du volume total d'acétonitrile sec. Le tube est fermé et mis à l'étuve à 60 °C pendant 30 minutes. Le mélange est ensuite laissé à refroidir. Après refroidissement du mélange, 450 µL d'acétonitrile sont ajoutés au mélange qui est à nouveau vortexé.

Le dosage peut alors avoir lieu et consiste à analyser la solution par chromatographie en phase gazeuse qui va permettre de séparer les triméthylsilanesters qui présenteront différents temps de rétention dans la colonne. Cette dernière peut être avantageusement une colonne SGE BPX5 (50 m x 0,22 mm x 0,25 µm) ou une colonne Agilent HP-5MS (50 m x 0,22 mm x 0,25 µm). Les conditions chromatographiques de température sont reprises au tableau 1.

Le four présente une température initiale de 60 °C. Le programme de température est repris dans le tableau 1.

**Tableau 1**

| | Montée en température (°C/min) | Température finale (°C) | Durée du maintien de la température finale (min) |
|---|---|---|---|
| 1 | 5,00 | 120 | 3,00 |
| 2 | 6,00 | 180 | 3,00 |
| 3 | 8,00 | 280 | 5,00 |

Le tableau 1 illustre le programme de température utilisé pour séparer les différents acides carboxylique urinaires. La température initiale du four est de 60 °C et est maintenu durant 3 minutes.

Une fois la séparation terminée, l'identification des acides carboxyliques urinaire est réalisée par spectrométrie de masse où la température du quadrupole présent dans le détecteur de masse s'élève à 150 °C et peut s'élever jusqu'à 200 °C.

Les résultats obtenus après dosage de l'échantillon d'urine sont repris dans le tableau 2.

**Tableau 2**

| La limite de détection est généralement de 1,0 mg/L, la limite de quantification est à 2 mg/L. | |
|---|---|
| Acides | Quantités (mmol/mol créatinine) |
| Lactate | < 6,6 |
| Benzoate | < 3,8 |
| furan-2,5-dicarboxylate | < 0 |
| Acétate de phényle | < 1,2 |
| Citramalate | < 0,81 |
| 2 OH-benzoate | < 0 |
| 2 OH-phénylacétate | < 0,43 |
| dl-3-phényllactate | < 0,08 |
| Tartarate | < 0,85 |
| 4-OH-benzoate | < 2 |
| 4-OH-phénylacétate | < 13,8 |
| tricarballylate | < 0,45 |
| p-OH-phénylpropionate | < 0,0 |
| 4-OH-phenyllactate | < 0,08 |
| hydrocafféate | < 0,13 |
| Acétate d'indole | < 0,28 |

Il est bien entendu que la présente invention n'est en aucune façon limitée aux formes de réalisations décrites ci-dessus et que bien des modifications peuvent y être apportées sans sortir du cadre des revendications annexées.

## Revendications

1. Procédé de dosage d'acides carboxyliques urinaires, comprenant les étapes de :
- amenée d'un volume prédéterminé d'un échantillon d'urine contenant des dérivés d'acides carboxyliques urinaires et des composants constitutifs d'urine,
- obtention d'un résidu sec desdits acides carboxyliques urinaires isolés desdits composants constitutifs d'urine, dans lequel ladite étape d'obtention dudit résidu sec comprend les étapes de :
∘ traitement dudit échantillon d'urine pour former lesdits acides carboxyliques urinaires,
∘ isolation desdits acides carboxyliques urinaires des composant constitutifs d'urine à l'aide d'un volume prédéterminé d'une phase d'élution, et
∘ évaporation dudit volume prédéterminé de ladite phase d'élution pour obtenir ledit résidu sec,
- estérification dudit résidu sec desdits acides carboxyliques urinaires par un mélange constitué de N-Méthyl-N-triméthylsilyltrifluoroacétamide (MSTFA) et de triméthylchlorosilane (TMCS) dans de l'acétonitrile sec anhydre pour former une solution comprenant des groupements triméthylsilanesters dérivés desdits acides carboxyliques urinaires,
- dosage par chromatographie en phase gazeuse couplée à une spectrométrie de masse desdits triméthylsilanesters pour obtenir une mesure de la quantité desdits triméthylsilanesters,
**caractérisé en ce que** l'étape d'isolation desdits acides carboxyliques est obtenue par adsorption sur de la terre de diatomées desdits acides carboxyliques et une élution desdits acides carboxyliques de la terre de diatomées par ledit volume prédéterminé de ladite phase d'élution contenant de l'acétate d'éthyle et du tert-butyl méthyl éther.

2. Procédé selon la revendication 1, ledit mélange constitué de N-Méthyl-N-triméthylsilyltrifluoroacétamide (MSTFA) et de triméthylchlorosilane (TMCS) dans de l'acétonitrile sec anhydre est présent en une quantité comprise entre 4 et 6 %, de préférence 5%, par rapport au volume total d'acétonitrile.

3. Procédé selon la revendication 1, dans lequel l'étape de traitement dudit échantillon d'urine comprend une addition d'une solution d'acide tropique diluée dans de l'acétonitrile et une addition d'acide chlorhydrique audit échantillon.

4. Procédé selon la revendication 3, dans lequel l'étape de traitement comprend l'addition de 50 µL de ladite solution d'acide tropique qui présente une concentration de 20 g/L et l'addition de 800 µL d'acide chlorhydrique à 37 % audit échantillon.

5. Procédé selon la revendication 1, dans lequel l'étape d'obtention dudit résidu sec comprend une étape de prétraitement, précédant ladite étape de traitement, qui consiste à prétraiter l'échantillon en présence d'azide de sodium et d'acide ascorbique.

6. Procédé selon la revendication 4, dans lequel ledit volume prédéterminé de ladite phase d'élution consiste en 25mL de ladite phase d'élution.

7. Procédé selon la revendication 1, dans lequel l'étape d'évaporation est réalisée sous azote à 37 °C.

8. Procédé selon l'une quelconque des revendications précédentes, dans lequel ladite étape d'estérification est réalisée durant une période de temps allant de 20 à 40 minutes, de préférence 30 minutes dans une étuve à une température supérieure à 50 °C, de préférence de 60°C.

## Patentansprüche

1. Verfahren zur Gehaltsbestimmung von Carbon-Harnsäuren, umfassend die Schritte:
- Zuleiten eines zuvor festgelegten Volumens einer Harnprobe, die Derivate von Carbon-Harnsäuren und konstituierende Bestandteile von Harn enthält,
- Erhalten eines trockenen Rückstands der von den konstituierenden Bestandteilen von Harn isolierten Carbon-Harnsäuren, wobei der Schritt des Erhaltens des trockenen Rückstands die Schritte umfasst:
∘ Behandlung der Harnprobe, um die Carbon-Harnsäuren zu bilden,
∘ Isolation der Carbon-Harnsäuren der konstituierenden Bestandteile von Harn mithilfe eines zuvor festgelegten Volumens einer Elutionsphase, und
∘ Verdampfen des zuvor festgelegten Volumens der Elutionsphase, um den trockenen Rückstand zu erhalten,
- Verestern des trockenen Rückstands der Carbon-Harnsäuren durch eine Mischung bestehend aus N-Methyl-N-trimethylsilyltrifluoracetamid (MSTFA) und Trimethylchlorsilan (TMCS) in wasserfreiem trockenem Acetonitril, um eine Lösung zu bilden, die von den Carbon-Harnsäuren abgeleitete Trimethylsilanestergruppen umfasst,
- Gehaltsbestimmung der Trimethylsilanester durch eine mit einer Massenspektrometrie gekoppelten Gaschromatographie, um ein Maß der Menge der Trimethylsilanester zu erhalten,
**dadurch gekennzeichnet, dass** der Isolationsschritt der Carbonsäuren durch Adsorption der Carbonsäuren an Diatomeenerde und eine Elution der Carbonsäuren von der Diatomeenerde durch das zuvor festgelegte Volumen der Elutionsphase, die Ethylacetat und tert-Butylmethylether enthält, erhalten wird.

2. Verfahren nach Anspruch 1, wobei die aus N-Methyl-N-trimethylsilyltrifluoracetamid (MSTFA) und Trimethylchlorsilan (TMCS) in trockenem wasserfreiem Acetonitril bestehende Mischung in einer Menge vorliegt, die zwischen 4 und 6 %, vorzugsweise 5 %, bezogen auf das Gesamtvolumen von Acetonitril enthalten ist.

3. Verfahren nach Anspruch 1, wobei der Behandlungsschritt der Harnprobe eine Zugabe einer in Acetonitril verdünnten Tropasäurelösung und eine Zugabe von Salzsäure zu der Probe umfasst.

4. Verfahren nach Anspruch 3, wobei der Behandlungsschritt die Zugabe von 50 µl der Tropasäurelösung, die eine Konzentration von 20 g/l aufweist, und die Zugabe von 800 µl Salzsäure von 37 % zu der Probe umfasst.

5. Verfahren nach Anspruch 1, wobei der Schritt des Erhaltens des trockenen Rückstands einen dem Behandlungsschritt vorausgehenden Vorbehandlungsschritt umfasst, der aus Vorbehandeln der Probe in Gegenwart von Natriumazid und Ascorbinsäure besteht.

6. Verfahren nach Anspruch 4, wobei das zuvor festgelegte Volumen der Elutionsphase aus 25 ml der Elutionsphase besteht.

7. Verfahren nach Anspruch 1, wobei der Verdampfungsschritt unter Stickstoff bei 37 °C durchgeführt wird.

8. Verfahren nach einem der vorstehenden Ansprüche, wobei der Veresterungsschritt während eines Zeitraums, der von 20 bis 40 Minuten, vorzugsweise 30 Minuten, reicht, in einer Wärmekammer bei einer Temperatur von mehr als 50 °C, vorzugsweise von 60 °C, durchgeführt wird.

## Claims

1. A method for assaying urinary carboxylic acids, comprising the steps of:
- supplying a predetermined volume of a urine sample containing derivatives of urinary carboxylic acids and constituent components of urine,
- obtaining a dry residue of said urinary carboxylic acids isolated from said constituent components of urine, wherein said step of obtaining said dry residue comprises the steps of:
∘ treating said urine sample to form said urinary carboxylic acids,
∘ isolating said urinary carboxylic acids from the constituent components of urine using a predetermined volume of an elution phase, and
∘ evaporating said predetermined volume of said elution phase to obtain said dry residue,
- esterifying said dry residue of said urinary carboxylic acids by a mixture consisting of N-Methyl-N-trimethylsilyltrifluoroacetamide (MSTFA) and trimethylchlorosilane (TMCS) in anhydrous dry acetonitrile to form a solution comprising trimethylsilane ester groups derived from said urinary carboxylic acids,
- assaying by gas chromatography coupled with mass spectrometry of said trimethylsilane esters to obtain a measurement of the quantity of said trimethylsilane esters,
**characterized in that** the step of isolating said carboxylic acids is obtained by adsorption on diatomaceous earth of said carboxylic acids and an elution of said carboxylic acids from the diatomaceous earth by said predetermined volume of said elution phase containing ethyl acetate and methyl tert-butyl ether.

2. The method according to claim 1, said mixture of N-Methyl-N-trimethylsilyltrifluoroacetamide (MSTFA) and trimethylchlorosilane (TMCS) in anhydrous dry acetonitrile is present in an amount between 4 and 6%, preferably 5%, based on the total volume of acetonitrile.

3. The method according to claim 1, wherein the step of treating said urine sample comprises an addition of a solution of tropic acid diluted in acetonitrile and an addition of hydrochloric acid to said sample.

4. The method according to claim 3, wherein the treatment step comprises adding 50µl of said tropic acid solution which has a concentration of 20g/L and adding 800µL of hydrochloric acid at 37% to said sample.

5. The method according to claim 1, wherein the step of obtaining said dry residue comprises a pre-treatment step, preceding said treatment step, which consists in pre-treating the sample in the presence of sodium azide and ascorbic acid.

6. The method according to claim 4, wherein said predetermined volume of said elution phase consists of 25mL of said elution phase.

7. The method according to claim 1, wherein the evaporation step is carried out under nitrogen at 37°C.

8. The method according to any of the preceding claims, wherein said esterification step is carried out over a period of time ranging from 20 to 40 minutes, preferably 30 minutes in an oven at a temperature above 50°C, preferably of 60°C.
